(19) Europäisches Patentamt
European Patent Office
Office européen des brevets

(11) Publication number: **0 038 645**
**B1**

# (12) EUROPEAN PATENT SPECIFICATION

(45) Date of publication of patent specification: **19.10.83**

(51) Int. Cl.³: **B 65 B 3/06**, A 45 D 33/00, A 45 D 33/02, B 65 D 83/06

(21) Application number: **81301504.7**

(22) Date of filing: **07.04.81**

## (54) Method of producing a cosmetic product containing a powder cake.

(30) Priority: **21.04.80 US 142447**

(43) Date of publication of application:
**28.10.81 Bulletin 81/43**

(45) Publication of the grant of the patent:
**19.10.83 Bulletin 83/42**

(84) Designated Contracting States:
**BE CH DE FR GB IT LI SE**

(56) References cited:
**DE - C - 512 744**
**GB - A - 923 077**
**US - A - 3 860 016**

(73) Proprietor: **KOLMAR LABORATORIES, INC.**
**123 Pike Street**
**Port Jervis New York 12771 (US)**

(72) Inventor: **Murphy, John H.**
**P.O. Box 162**
**Matamoras Pennsylvania 18336 (US)**
Inventor: **Brodzinski, John J.**
**P:O: Box 2190**
**Milford Pennsylvania 18337 (US)**
Inventor: **Horton, Donald D.**
**P:O: Box 496**
**Glen Spey New York 12737 (US)**

(74) Representative: **Brown, David Alan et al,**
**MATHYS & SQUIRE 10 Fleet Street**
**London EC4Y 1AY (GB)**

**EP 0 038 645 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Method of producing a cosmetic product containing a powder cake

This invention relates to a method of producing a cosmetic product, said product including a casing having a bottom surface and an open top and a cover to enclose the open top, said casing defining a cavity.

Pressed powders, eye shadow, blushes and face powders currently on the market are produced by compressing loose powder into a metal pan using direct pressure. The pan containing the pressed powder cake is then glued into the compact, which is the primary container.

In the conventional process the pressure used in the pressing operation must be maintained within precise limits. If the pressure is too great, the metal pan may be distorted and must be discarded. In addition, utilizing a high pressure results in a cake which lacks the desired "pay off" characteristics. On the other hand, if the pressure is too low, the pressed cake will lack cohesive strength. Thus, certain powder compositions cannot successfully be used as a pressed powder cake because the pressure required to obtain the necessary cohesive strength will either distort the metal pan or produce a cake that is so hard that it lacks "pay-off".

As a further problem, aerobic bacteria may frequently contaminate the pressed pans, so that the pans must be sterilized before final assembly either by heat or other techniques. This further adds to the cost in producing the pressed powder product.

Accordingly it is an object of the present invention to provide a method of producing a cosmetic product containing a powder composition that is moulded directly in the casing without the use of a separate metal pan.

The method of this invention is characterised in that said bottom surface has an opening communicating with the cavity, and in that the method comprises the steps of inverting the casing so that the opening is exposed and positioning the open top against a supporting surface, preparing a liquid slurry comprising a finely divided cosmetically acceptable filler, a fatty alcohol containing 12 to 22 carbon atoms in the molecule and a liquid volatile carrier, introducing the slurry into the opening to fill the cavity and form a moulded cake against said supporting surface, drying the moulded cake to evaporate the liquid volatile carrier and thereby to cause a corresponding migration of the fatty alcohol toward the exposed surface of the moulded cake, and closing the opening in the bottom surface with a backing member and the open top with the cover.

In the method of the invention, the migration of the fatty alcohol results in an increased concentration of the fatty alcohol at the rear exposed surface of the powder cake which adds strength and support to the final product.

With the method of the invention, the powder cake is formed directly in the casing without the use of a metal pan, as has been required with prior art methods. By eliminating the use of the metal pan, the design configurations of the cake are greatly increased because the configurations are not restricted to the design of the metal pan.

The invention also provides a substantial cost reduction since the metal pans are eliminated and the labour and assembly operations are also greatly reduced.

By using the slurry technique for forming the powder cake, finely divided materials, not capable of being successfully compressed, can be used to form the cake.

The micro-biological problems are minimised by the invention, since the product is poured and dried at elevated temperatures which discourages microbial contamination. In addition, the only surface of the cake exposed to the air is at the bottom of the casing, which is subsequently covered and is never used by the consumer.

The invention also includes a cosmetic product comprising a casing having a bottom surface and an open top, said casing defining at least one cavity containing a moulded powder cake, and a cover to enclose the open top of said casing, characterised in that the bottom surface of said casing has an opening communicating with said cavity, the moulded powder cake extending within said opening and being composed of a cosmetic powdered material, a fatty alcohol containing from 12 to 22 carbon atoms in the molecule, cosmetic colouring material and a residual amount up to 2% by weight of an evaporable liquid carrier, and the portion of the cake in the region of the opening having an increased concentration of said fatty alcohol to provide increased toughness for the cake in said region.

The invention will now be described, by way of example, with reference to the accompanying drawings, in which:

FIG. 1 is a perspective view of a compact to be used in the method of the invention with the cover shown in the open position:

FIG. 2 is a bottom view of the completed compact with parts broken away;

FIG. 3 is a section taken along line 3—3 of FIG. 2;

FIG. 4 is a diagrammatic perspective view of the casing of a further embodiment of the invention;

FIG. 5 is a diagrammatic cross-section through a compact in accordance with another embodiment of the invention.

FIGS. 6 and 7 illustrate alternative arrangements of openings in the bottom wall of a compact in accordance with the invention;

FIG. 8 is a perspective view of a compact in accordance with yet another embodiment of the invention;

FIG. 9 is a diagrammatic cross-section through the casing of a compact in accordance with the invention, showing a modified form of the openings in the bottom wall of the casing;

FIG. 10 is a perspective view of the casing of a compact in accordance with yet another embodiment of the invention;

FIG. 11 is a section on line 11—11 of FIG. 10, showing the casing inverted against a filling surface; and

FIG. 12 is an exploded view of a compact in accordance with yet a further embodiment of the invention.

Description of the Preferred Embodiment

Fig. 1 illustrates a cosmetic product, such as a compact 1, produced in accordance with the method of the invention. The compact 1 includes a casing 2 and a cover 3 which is hinged to the casing. As shown in Fig. 1, a mirror 4 can be mounted on the underside of the cover.

The casing 2 includes an upper surface 5 and a back surface 6. The upper surface is formed with a shallow tray 7 which normally would receive an applicator, not shown, for applying the powder cake to the skin.

The casing defines a central cavity 8 and a grid 9, which is formed integrally with the back surface 6, extends across the rear portion of the cavity 8. A molded powder cake 10 is disposed within the cavity 8 and extends within the openings in the grid 9.

Mounted over the upper surface of the cake 10 is a purity seal 11 which can be formed of plastic or paper. At the time of use, the purity seal 11 will be removed by the consumer.

The cavity 8 can be closed off on the back surface 6 of the casing, by a back plate 12 which is snapped into place on the back surface 6. As shown in the drawings, the back surface 6 can be provided with a series of outwardly extending projections 13 which are snap fitted within openings in the plate 12. Alternatively, a label or small decorative plate can be attached to the back surface 6 over the cavity 8 by an adhesive, as a substitute for the back plate 12, or in some cases, the grid 9 will be covered and will be exposed.

In general, the powder cake is prepared from a slurry having the following formulation in weight per cent:

| | |
|---|---|
| Finely divided inert filler | 70%—10% |
| Fatty alcohol | 5%—30% |
| Volatile siloxane | 25%—60% |
| Cosmetic colouring materials | 1%—35% |

The finely divided filler can take the form of materials such as aluminium hydroxide, kaolin, talc, mica, corn starch, calcium carbonate, silicon dioxide, calcined clay, barium sulfate, aluminium oxide, aluminium silicate, and the like.

The fatty alcohols are miscible with the siloxane and serve as a binder for the system and contain from 12 to 22 carbon atoms in the molecule. The fatty alcohol can take the form of cetyl alcohol, stearyl alcohol, and the like.

The volatile siloxane is normally a liquid at room temperature. Chemically it is composed primarily of two cyclic components: $D_4$ cyclodimethicone and $D_5$ cyclodimethicone. The $D_4$ component represents the majority with the $D_5$ being a minor constitutent. Chemically $D_4$ cyclodimethane may be symbolically written as:

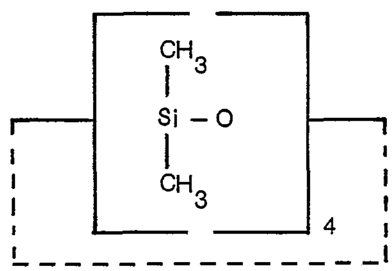

and $D_5$ cyclodimethicone may be symbolically written as:

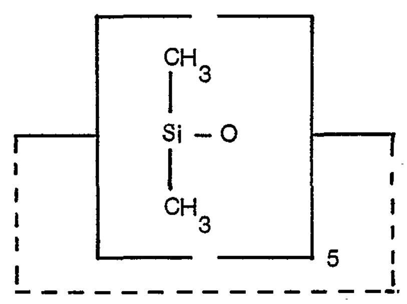

The cosmetically acceptable coloring materials can take the form of titanium dioxide, ferric ferrocyanide or ferric ammonium ferrocyanide, iron oxides, ultramarines, chromium oxide, chromium hydroxide, pearlescents, organic dyes, and lakes.

In addition to the above ingredients, the slurry can also contain small amounts, up to 2% by weight, of a fatty acid ester containing from 12 to 22 carbon atoms, such as iso-propyl myristate or isopropyl palmitate, which prevents dusting of the cake; and/or magnesium stearate which aid in preventing glazing of the cake; and/or a preservative such as methyl paraben or propyl paraben; or perfumes.

To prepare the product of the invention, a purity seal 11 is placed on the upper surface 5 of the casing 2, covering the cavity 8, and the cover 3 is then closed and the compact inverted so that the back surface 6 faces upwardly.

A liquid slurry is then prepared by dispersing the fatty alcohol in the liquid siloxane at a temperature generally in the range of 60°C to 70°C. The remaining ingredients, such as the powdered filler, coloring materials, and other

additives are then mixed into the liquid dispersion to provide the slurry. The slurry, at a temperature in the range of about 50°C to 60°C, is then poured by gravity into the cavity 8 in the back surface 6 of the compact to fill the cavity and the spaces within the grid. On cooling to a temperature below 45°C, the slurry will solidify to form a solid cake.

Subsequently, the compact is placed in a drying oven at a temperature of about 40°C for 60 hours to evaporate the siloxane from the cake, so that the dried cake has a residual siloxane content of less than 2% by weight. While it is possible to evaporate the entire siloxane content, it is normally uneconomical to go beyond the 2% level although this can be accomplished by variations in relative humidity and pressure.

During drying, the siloxane will vaporize and the vapor is discharged from the cake through the exposed open bottom of the cavity. The vapor flow will cause a corresponding migration of the fatty alcohol toward the exposed surface, resulting in an increased concentration of the fatty alcohol in the vicinity of the exposed surface. The increased concentration of fatty alcohol will provide greater toughness for the cake in the region of grid 9 to aid in retaining the cake within the cavity.

After drying of the cake to evaporate the siloxane, the plastic bottom plate 12 can be snapped into position and the compact is ready for shipment.

When the consumer opens the compact, the purity seal 11, which serves as a mold for the powder cake 10, is removed and a smooth uniform surface is exposed for use. As the powder cake 10 is molded directly into the compact, it eliminates the use of the conventional metal pan and correspondingly results in a cost reduction.

Instead of moulding the powder cake 10 against the purity seal 11, the casing 2 could be placed against a separate surface, such as a removable sealing matt, which is removed before the cover 3 is closed. Alternatively, the cover 3 could be provided with a suitable inner face against which the cake can be moulded, the cover being closed before the slurry is introduced into the casing. Where the cake 10 is moulded against the purity seal 11 or a separate surface, it is preferred, for ease of manufacture, that the cover be hinged to the casing in such a manner that it can open through 180°, to enable the inverted casing and cover to be placed flat to ensure a seal between the edge of the cavity 8 and the filling surface. Alternatively, the cover 3 may be detachable from the casing.

By utilizing the slurry technique with the evaporable siloxane carrier, hard-to-press powdered materials can be satisfactorily employed in the cake. The evaporation of the siloxane carrier increases the porosity of the solidified cake, resulting in increased "pay-off" for the cake.

The cavity 8 and the moulded cake can be formed with a wide variety of configurations which are not possible when using a metal pan, as in the prior art. For example, the cake 10 can be formed in a irregular or complex shape, such as the leaf design illustrated in Figure 4.

Moreover, the cake can be formed with a contoured surface to provide a three-dimensional shape to its upper surface. For example, as shown diagrammatically in Figure 5, the cover 3 of the compact may have an inner face 15 against which the cake 10 is moulded, the face 15 being formed with recesses 16. When the slurry is introduced into the inverted casing 2, the cover 3 being closed, it flows into the recesses 16, so that the finished cake 10 has embossed portions 17 standing out in relief from the surface of the cake. The lid could also be formed with projections on its inner surface 15, producing corresponding depressions in the moulded cake 10. Quite complex shapes can be formed in this manner on the surface of the cake. The shapes could also be formed by moulding the cake against a suitably shaped purity seal or separate filling surface.

It will be appreciated that the casing 2 could take many different forms. In particular, the grid 9 through which the slurry is introduced into the casing could be replaced by other arrangements of openings. For example, Figure 6 shows a modified form of casing in which the lower wall 18 through which the slurry is introduced is formed with a regular array of circular holes 19. Figure 7 shows a further modification in which the bottom wall 18 of the casing is formed with separate groups of holes, through which slurry can be directed to separate areas of the casing. For example, the casing may have two separate cavities 25 and 26, as shown in Figure 8. The side walls 22 and 23 of the casing 2 extend somewhat beyond the bottom wall 18, to form a well 24 into which the slurry is introduced during the filling process. This helps to direct the slurry into the holes in the bottom wall, and, in the embodiment of Figure 8 enables both cavities 25 and 26 to be filled in a single operation.

The openings through which the slurry is introduced into the casing may have a greater cross-section at the outer face of the bottom wall than the cross-section at the inner face, to assist in locking the moulded cake into the casing. For example, Figure 9 is a section through an inverted casing 2, in which the dimensions of the openings 27 and the thickness of the bottom wall have been exaggerated for the sake of clarity. Each opening 27 has an inner portion 28 of uniform cross-section leading from the inner face 29 of the bottom wall and an outer portion 30 which is tapered, increasing in cross-section to the outer face 31 of the bottom wall. The faces 32 of the outer portion 30 are inclined at an angle to the faces 29, 31 of the bottom wall, so that the cake 10 is

keyed to the bottom wall.

As shown in Figure 9 slurry forming the cake 10 is filled to a level 33 above the outer face 31 of the bottom wall, so that the cake 10 is interlocked with the bottom wall. A back-plate 12, of plastics or cardboard, may be fixed to the underside of the casing 2 as in the first-described embodiment, the back-plate engaging a peripheral ledge 34 at a level spaced from that of outer face 31 of the bottom wall.

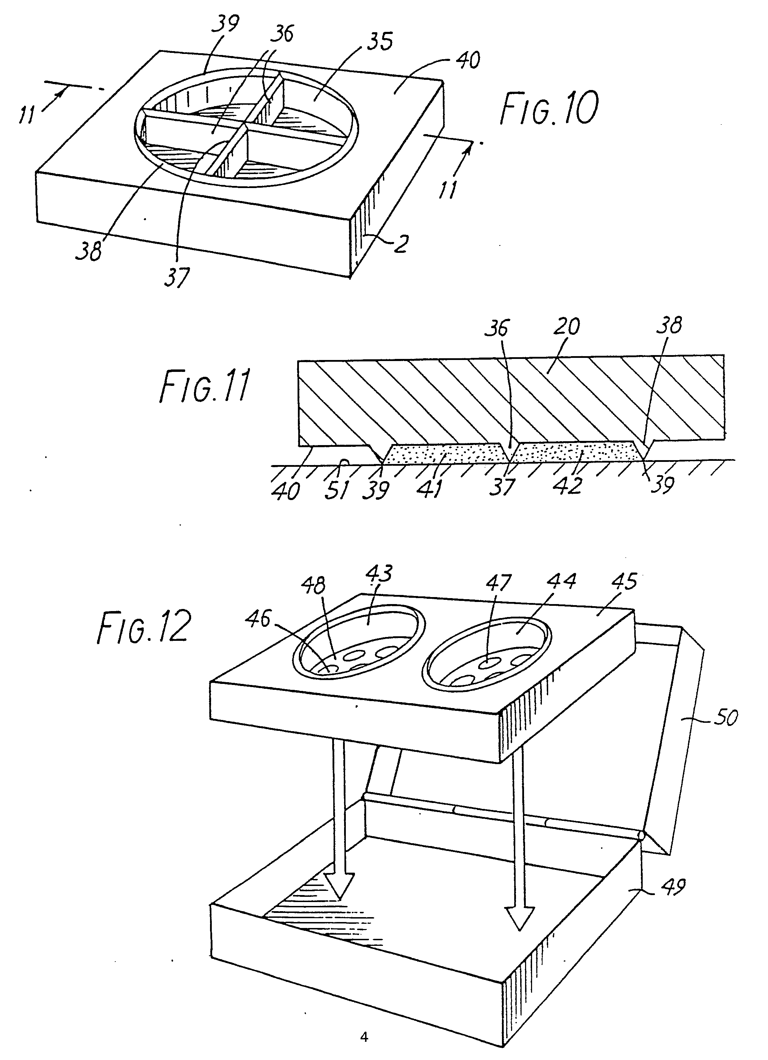

In the embodiment of Figure 10, the cavity 35 in the casing 2 is divided into four areas by vertical partition members 36, which may be formed integrally with the casing. The partition members 36 extend slightly above the upper face 40 of casing 2, to form narrow sealing edges 37, and a raised rim 38 extending around the edge of the cavity 35 similarly provides a sealing edge 39. When the casing is inverted against a filling surface 51, as shown in Figure 11, the sealing edges 37 and 39 engage the surface 41 so as to separate the four areas of the cavity from one another. This enables powder cakes of different colours to be introduced into the separate areas. Thus slurry 41, mixed with a suitable pigment, can be introduced into one area through appropriately positioned holes in the bottom wall of the cavity, whilst slurry 42 containing a different pigment can be introduced into another area. By using multiple nozzles, the different slurries can be introduced simultaneously. It will be appreciated that partition members of various different configuration could be used to divide the cavity in the casing into two or more areas of various shapes.

In the embodiment shown in Figure 12, cavities 43 and 44 are formed in a separate insert 45. The powder cake is formed in each cavity by introducing slurry into the cavities through openings 46 and 47 in the bottom wall 48 of the inverted insert, the cakes being moulded against a filling surface as in the previously described embodiments. The insert is then fitted into a separately formed container 49 having a hinged lid 50. The container 49 may be a standard container, and the insert 45 may be of injection moulded or vacuum formed plastics.

**Claims**

1. A method of producing a cosmetic product, said product including a casing (2, 45) having a bottom surface and an open top and a cover (3, 50) to enclose the open top, said casing defining at least one cavity (8, 25, 26, 43, 44) to contain a powder cake (10), characterised in that said bottom surface has an opening communicating with the cavity (8, 25, 26, 43, 44) and in that the method comprises the steps of inverting the casing (2, 45) so that the opening is exposed and positioning the open top against a supporting surface, preparing a liquid slurry comprising a finely divided cosmetically acceptable filler, a fatty alcohol containing 12 to 22 carbon atoms in the molecule and a liquid volatile carrier, introducing the slurry into the opening to fill the cavity (8, 25, 26, 43, 44) and form a moulded cake (10) against said supporting surface, drying the moulded cake (10) to evaporate the liquid volatile carrier and thereby to cause a corresponding migration of the fatty alcohol toward the exposed surface of the moulded cake, and closing the opening in the bottom surface with a backing member (12) and the open top with the cover (3, 50).

2. A method of claim 1, characterised by the step of positioning a layer of removable sealing matt in the cavity (8) against the supporting surface prior to introducing the slurry into the cavity, said slurry being moulded against said material, and removing the layer from contact with said moulded cake prior to closing the open top of the casing (2) with said cover (3).

3. The method of claim 1 or claim 2, characterised by the step of maintaining the temperature of the slurry in the range of 50°C to 60°C when it is introduced into the cavity (8, 25, 26, 43, 44).

4. The method as claimed in any preceding claim, characterised in that the step of introducing the slurry comprises pouring the slurry by gravity into the cavity.

5. The method as claimed in any preceding claim, characterised in that said slurry is composed of 3% to 75% by weight of said finely divided powder material, 4% to 12% by weight of said fatty alcohol, 1% to 35% by weight of cosmetic colouring materials, and 20% to 50% by weight of a liquid volatile siloxane, as said carrier.

6. The method of claim 5, characterised in that the step of evaporating the siloxane is accomplished by contacting the portion of the moulded cake (10) exposed through said opening with a gas while maintaining said moulded cake (10) in contact with said supporting surface.

7. The method as claimed in any preceding claim, characterised in that the slurry is moulded against a supporting surface (15) having a three-dimensional shape.

8. The method as claimed in any preceding claim, characterised in that the supporting surface (15) is an inner surface of the cover (3), and in which the cover (3) is closed before introduction of the slurry into the cavity.

9. The method as claimed in any preceding claim, characterised in that the casing (2) has a plurality of cavities (25, 26) and slurry is introduced simultaneously into each of the cavities (25, 26).

10. The method as claimed in any preceding claim characterised by introducing slurry through one or more openings (27) in the bottom wall of the casing, each opening having a cross-sectional area which is greater at the outer face (31) of the bottom wall that at the

inner face (29).

11. The method as claimed in any preceding claim, characterised in that said cavity is divided by partition members (36) into a plurality of separate volumes, and in that slurries having different characteristics are introduced into the respective volumes.

12. The method as claimed in claim 11, characterised by introducing slurries (41, 42) of different colours into the respective volumes.

13. The method as claimed in any preceding claim, characterised by moulding the cake into a casing (45) and subsequently inserting the casing into a separate container (49) to which said cover (50) is attached.

14. A cosmetic product, comprising a casing (2, 45) having a bottom surface and an open top, said casing defining at least one cavity (8, 25, 26, 43, 44) containing a moulded powder cake (10) and a cover (3, 50) to enclose the open top of said casing, characterised in that the bottom surface of said casing (2, 45) has an opening communicating with said cavity, the moulded powder cake (10) extending within said opening and being composed of a cosmetic powdered material, a fatty alcohol containing from 12 to 22 carbon atoms in the molecule, cosmetic colouring materials, and a residual amount up to 2% by weight of an evaporable liquid carrier, and the portion of the cake (10) in the region of the opening having an increased concentration of said fatty alcohol.

15. A product as claimed in claim 14, characterised in that a grid (9) is disposed within the opening, said cake extending within the spaces in said grid (9).

16. A product as claimed in claim 14 or claim 15, characterised in that a backing member is disposed on said bottom surface and extending across the opening to cover the same.

17. A product as claimed in any one of claims 14 to 16, characterised in that a sealing sheet (11) is disposed between the casing (2) and the cover (3) and extending across the cavity.

**Revendications**

1. Un procédé pour la production d'un produit cosmétique, ledit produit comprenant une enveloppe (2, 45) possédant une surface de fond et une partie supérieure ouverte et un couvercle (3, 50) pour fermer la partie supérieure ouverte, ladite enveloppe délimitant au moins une cavité (8, 25, 26, 43, 44) destinée à contenir une masse compacte de poudre (10), caractérisé en ce que ladite surface de fond possède une ouverture communiquant avec la cavité (8, 25, 26, 43, 44), et en ce que le procédé comprend les étapes de retournement de l'enveloppe (2, 45) de sorte que l'ouverture est exposée, et de positionnement de la partie supérieure ouverte contre une surface de support, de préparation d'une pâte liquide comprenant un remplisseur finement divisé acceptable d'un point de vue cosmétique, un alcool gras contenant 12 à 22 atomes de carbone dans la molécule et un porteur liquide volatile, d'introduction de la pâte dans l'ouverture pour remplir la cavité (8, 25, 26, 43, 44) et former une masse compacte moulée (10) contre ladite surface de support, de séchage de la masse compacte moulée (10) pour évaporer le véhicule volatil liquide et provoquer ainsi une migration correspondante de l'alcool gras vers la surface exposée de la masse compacte moulée, et de fermeture de l'ouverture de la surface de fond avec une pièce de soutien (12) et de la partie supérieure avec le couvercle (3, 50).

2. Un procédé selon la revendication 1, caractérisé par l'étape de positionnement d'une couche d'un matériau d'obturation enlevable dans la cavité (8) contre la surface de support avant d'introduire la pâte dans la cavité, ladite pâte étant moulée contre ledit matériau, et d'enlèvement de la couche de son contact avec ladite masse compacte moulée avant de fermer la partie supérieure ouverte de l'enveloppe (2) avec ledit couvercle (3).

3. Un procédé selon les revendications 1 ou 2, caractérisé par l'étape de maintien de la température de la pâte dans un intervalle compris entre 50°C et 60°C, quand elle est introduite dans la cavité (8, 25, 26, 43, 44).

4. Un procédé selon n'importe laquelle des revendications précédentes, caractérisé en ce que l'étape d'introduction de la pâte comprend le fait de verser la pâte par gravité dans la cavité.

5. Un procédé selon n'importe laquelle des revendications précédentes, caractérisé en ce que ladite pâte est composée de 3% à 76% en poids dudit matériau un poudre finement divisé, de 4% à 12% en poids dudit alcool gras, de 1% à 35% en poids des matériaux colorants cosmétiques, et de 20% à 50% en poids d'un siloxane volatile liquide, comme ledit véhicule.

6. Un procédé selon la revendication 5, caractérisé en ce que l'étape d'évaporation du siloxane est acomplie en mettant en contact la partie de la masse compacte moulée (10) exposée par ladite ouverture avec un gaz tout en maintenant ladite masse compacte moulée (10) en contact avec la surface de support.

7. Un procédé selon n'importe laquelle des revendications précédentes, caractérisé en ce que la pâte est moulée contre une surface de support (15) possédant une forme tri-dimensionnelle.

8. Une procédé selon n'importe laquelle des revendications précédentes, caractérisé en ce que la surface de support (15) est une surface intérieure de couvercle (3), et dans lequel le couvercle (3) est fermé avant l'introduction de la pâte dans la cavité.

9. Un procédé selon n'importe laquelle des revendications précédentes, caractérisé en ce que l'enveloppe (2) possède plusieurs cavités (25, 26) et que la pâte est introduite simultanément dans chacune des cavités (25, 26).

10. Un procédé selon n'importe laquelle des

revendications prédédentes, caractérisé par l'introduction de la pâte à travers une ou plusieurs ouvertures (27) dans la paroi de fond de l'enveloppe, chaque ouverture ayant une surface de section transversale qui est plus grande sur sa face extérieure (31) de la paroi de fond que sur sa face intérieure (29).

11. Un procéde selon n'importe laquelle des revendications précédentes, caractérisé en ce que ladite cavité est divisée par des cloisons (36) en plusieurs volumes distincts, et en ce que des pâtes ayant des caractéristiques différentes sont introduites dans les volumes respectifs.

12. Un procédé selon la revendication 11, caractérisé par l'introduction de pâtes (41, 42) de différentes couleurs dans les volumes respectifs.

13. Un procédé selon n'importe laquelle des revendications précédentes, caractérise par le moulage de la masse compacte dans une enveloppe (45) et ensuite, par l'insertion de l'enveloppe dans un récipient distinct (49) auquel ledit couvercle (50) est attaché.

14. Un produit cosmétique, comprenant une enveloppe (2, 45) possédant une surface de fond et une partie supérieure ouverte, ladite enveloppe délimitant au moins une cavité (8, 25, 26, 43, 44) contenant une masse compacte de poudre moulée (10) et un couvercle (3, 50) pour fermer la partie supérieure de ladite enveloppe, caractérisé en ce que la surface de fond de ladite veloppe (2, 45) possède une ouverture communiquant avec ladite cavité, la masse compacte de poudre moulée (10) se déployant par ladite ouverture et étant composée d'un matériau en poudre cosmétique, d'un alcool gras contenant de 12 à 22 atomes de carbone dans la molécule, de matériaux cosmétiques colorants, et d'une quantité résiduelle pouvant atteindre 2% en poids d'un véhicule liquide évaporable, et la partie de la masse compacte (10) dans la zone de l'ouverture ayant une concentration croissante dudit alcool gras.

15. Un produit selon la revendication 14, caractérisé en ce qu'une grille (9) est disposée dans l'ouverture, ladite masse compacte se déployant à l'intérieur des espaces de ladite grille (9).

16. Un produit selon les revendications 14 ou 15, caractérisé en ce qu'une pièce de soutien est disposée sur ladite surface de fond et s'étend en travers de l'ouverture pour la couvrir.

17. Un produit selon n'importe laquelle des revendications 14 à 16, caractérisé en ce qu'une feuille d'obturation (11) est disposée entre l'enveloppe (2) et le couvercle (3) et s'étend en travers de la cavité.

## Patentansprüche

1. Verfahren zur Herstellung eines kosmetischen Produkts, wobei dieses Produkt ein Gehäuse (2, 45) mit einer Bodenfläche und einer offenen Oberseite und einen Deckel (3, 50) zum Umschließen der offenen Oberseite umfaßt, und das Gehäuse mindestens einen Hohlraum (8, 25, 26, 43, 44) zur Aufnahme eines Puderkuchens (10) umfaßt, dadurch gekennzeichnet, daß die Bodenfläche eine Öffnung, in Verbindung mit dem Hohlraum (8, 25, 26, 43, 44), aufweist, und daß das Verfahren folgende Stufen umfaßt: Umdrehen des Gehäuses (2, 45) derart, daß die Öffnung frei liegt und die offene Oberseite gegen eine Stützfläche zu liegen kommt, Bereiten einer flüssigen Aufschlämmung, die einen feinverteilten, kosmetisch brauchbaren Füllstoff, einen Fettalkohol mit 12 bis 22 Kohlenstoffatomen im Molekül und einen flüssigen, flüchtigen Träger enthält, Einbringen der Aufschlämmung in die Öffnung, um den Hohlraum (8, 25, 26, 43, 44) zu füllen und einen geformten Kuchen (10) gegen die Stützfläche zu bilden, Trocknen des geformten Kuchens (10) zur Verdampfung des flüssigen, flüchtigen Trägers und hierdurch eine entsprechende Wanderung des Fettalkohols zu der freiliegenden Oberfläche des geformten Kuchens zu bewirken, und Schließen der Öffnung in der Bodenfläche mit einem Rückseitenteil (12) und der offenen Oberseite mit dem Deckel (3, 50).

2. Verfahren nach Anspruch 1, gekennzeichnet durch eine Stufe, bei der eine Schicht aus einer entfernbaren Dichtungsmatte in den Hohlraum (8) gegen die Stützfläche vor dem Einführen der Aufschlämmung in den Hohlraum gelget wird, wobei die Aufschlämmung gegen dieses Material geformt wird, und Entfernen der Schicht aus dem Kontakt mit dem geformten Kuchen, vor dem Schließen der offenen Oberseite des Gehäuses (2) mit dem Deckel (3).

3. Verfahren nach Anspruch 1 oder 2, gekennzeichnet durch eine Stufe, bei der die Temperatur der Aufschlämmung in dem Bereich von 50°C bis 60°C gehalten wird, wenn sie in den Hohlraum (8, 25, 26, 43, 44) eingebracht wird.

4. Verfahren nach einem der vorhergehenden Ansprüche, dadurch gekennzeichnet, daß die Stufe des Einbringens der Aufschlämmung das Gießen der Aufschlämmung durch Schwerkraft in den Hohlraum umfaßt.

5. Verfahren nach einem der vorhergehenden Ansprüche, dadurch gekennzeichnet, daß die Aufschlämmung aus 3 bis 75 Gew.-% des feinverteilten Pulvermaterials, 4 bis 12 Gew.-% des Fettalkohols, 1 bis 35 Gew.-% an kosmetischen Färbematerialien und 20 bis 50 Gew.-% eines flüssigen, flüchtigen Siloxans als Träger besteht.

6. Verfahren nach Anspruch 5, dadurch gekennzeichnet, daß die Stufe des Verdampfens des Siloxans durchgeführt wird durch Kontakt des Teils, des geformten Kuchens (10), der durch die Öffnung freigelegt wird, mit einem Gas, wobei der geformte Kuchen (10) in Kontakt mit der Stützfläche gehalten wird.

7. Verfahren nach einem der vorhergehenden Ansprüche dadurch gekennzeichnet, daß die Aufschlämmung gegen eine Stützfläche (15) mit dreidimensionaler Form geformt wird.

8. Verfahren nach einem der vorhergehenden Ansprüche, dadurch gekennzeichnet, daß die Stützfläche (15) eine innere Fläche des Deckels (3) ist, und daß der Deckel (3) vor dem Einführen der Aufschlämmung in den Hohlraum geschlossen wird.

9. Verfahren nach einem der vorhergehenden Ansprüche, dadurch gekennzeichnet, daß das Gehäuse (2) mehrere Hohlräume (25, 26) aufweist, und die Aufschlämmung gleichzeitig in jeden der Hohlräume (25, 26) eingeführt wird.

10. Verfahren nach einem der vorhergehenden Ansprüche dadurch gekennzeichnet, daß die Aufschlämmung durch eine oder mehrere Öffnungen (27) in der Bodenwandung des Gehäuses eingeführt wird, wobei jede Öffnung eine Querschnittsfläche aufweist, die an der äußeren Fläche (31) der Bodenwandung größer ist als an der inneren Fläche (29).

11. Verfahren nach einem der vorhergehenden Ansprüche, dadurch gekennzeichnet, daß der Hohlraum durch Teilglieder (36) in mehrere getrennte Volumen aufgeteilt ist, und daß Aufschlämmungen mit underschiedlichen Charakteristika in die jeweiligen Volumen eingeführt werden.

12. Verfahren nach Anspruch 11, dadurch gekennzeichnet, daß Aufschlämmungen (41, 42) verschiedener Farben in die jeweiligen Volumen eingeführt werden.

13. Verfahren nach einem der vorhergehenden Ansprüche, dadurch gekennzeichnet, daß der Kuchen in ein Gehäuse (45) geformt wird, und anschließend das Gehäuse in einen getrennten Behälter (49) eingeführt wird, an das der Deckel (50) befestigt ist.

14. Kosmetisches Produkt, enthaltend ein Gehäuse (2, 45) mit einer Bodenfläche und einer offenen Oberseite, wobei das Gehäuse mindestens einen Hohlraum (8, 25, 26, 43, 44) definiert, der einen geformten Puderkuchen (10) enthält, und einem Deckel (3, 50), um die offene Oberseite des Gehäuses zu umschließen, dadurch gekennzeichnet, daß die Bodenfläche des Gehäuses (2, 45) eine Öffnung in Verbindung mit dem Hohlraum aufweist, sich der geformte Pulverkuchen (10) innerhalb der Öffnung erstreckt und aus einem kosmetischen, pulverförmigen Material, einem Fettalkohol mit 12 bis 22 Kohlenstoffatomen im Molekül, kosmetischen Färbematerialien und einer Restmenge von bis zu 2 Gew.-% eines verdampfbaren, flüssigen Trägers besteht, und der Teil des Kuchens (10) im Gebiet der Öffnung eine erhöhte Konzentration des Fettalkohols aufweist.

15. Produkt nach Anspruch 14, dadurch gekennzeichnet, daß ein Gitter (9) in der Öffnung angeordnet ist, und daß sich der Kuchen in die Öffnungen dieses Gitters (9) erstreckt.

16. Produkt nach Anspruch 14 oder Anspruch 15, dadurch gekennzeichnet, daß ein Rückseitenteil auf der Bodenfläche angeordnet ist und sich über die Öffnung erstreckt, um diese zu bedecken.

17. Produkt nach einem der Ansprüche 14 bis 16, dadurch gekennzeichnet, daß eine Verschluß-Folie (11) zwischen dem Gehäuse (2) und dem Deckel (3) angeordnet ist und sich über den Hohlraum erstreckt.

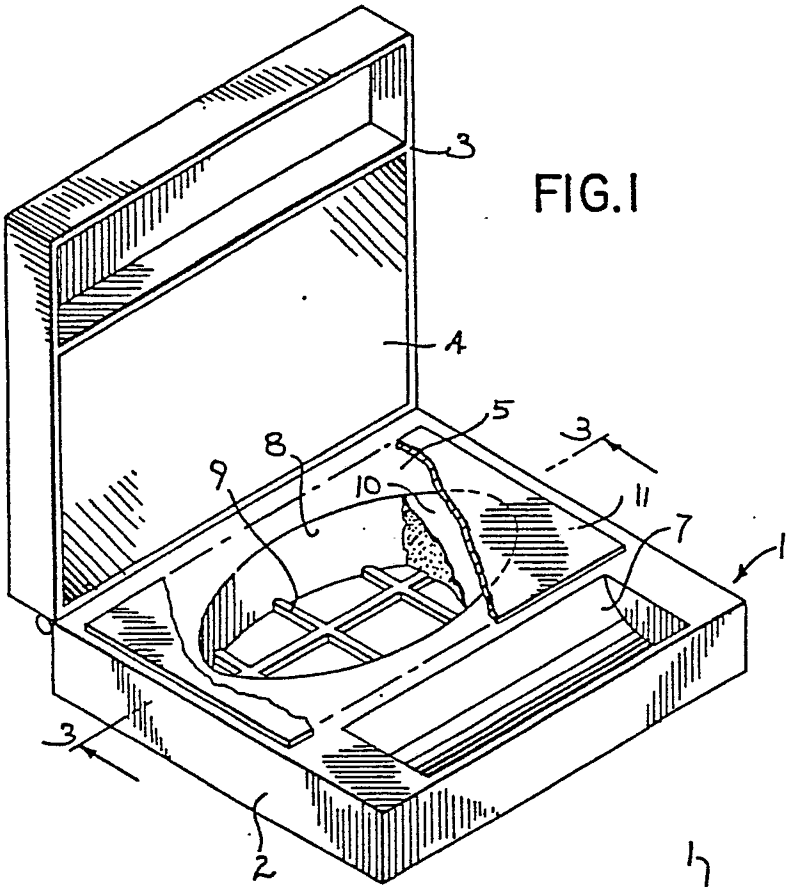


FIG. 1

FIG. 2

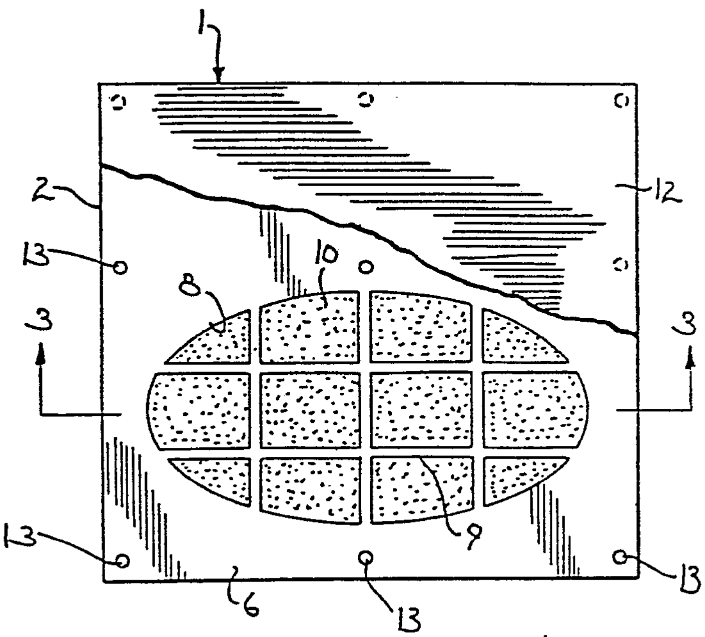


FIG. 3

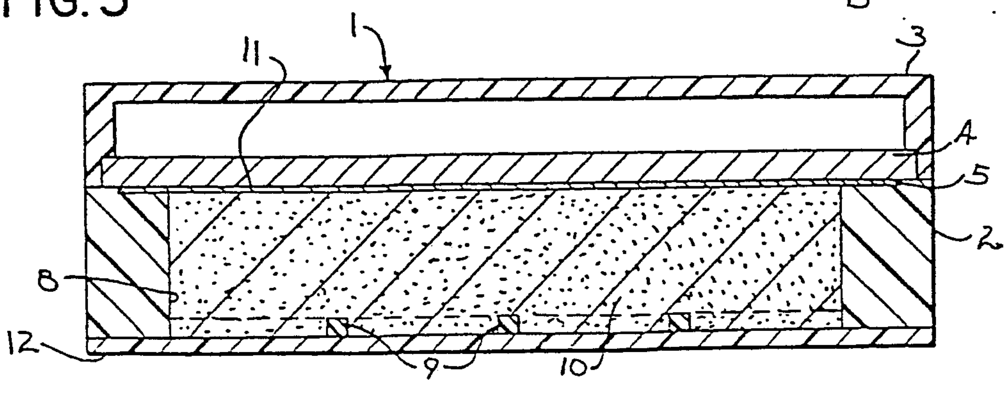

FIG.4

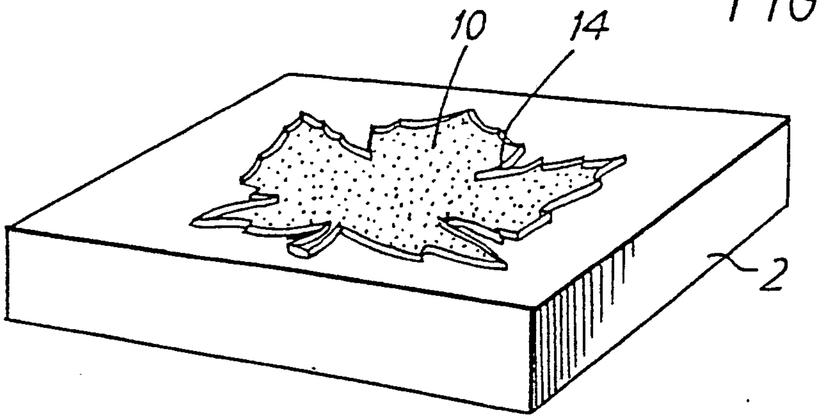


FIG.5

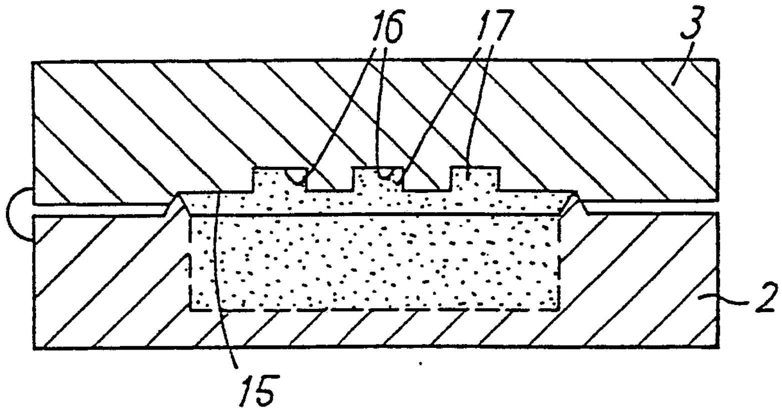


FIG.6

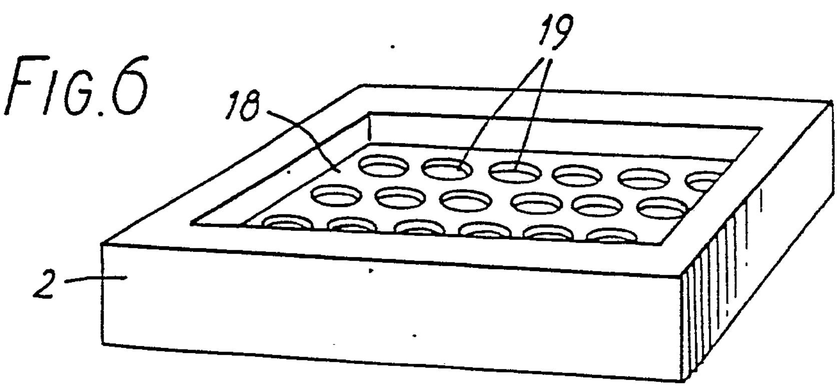


FIG.7

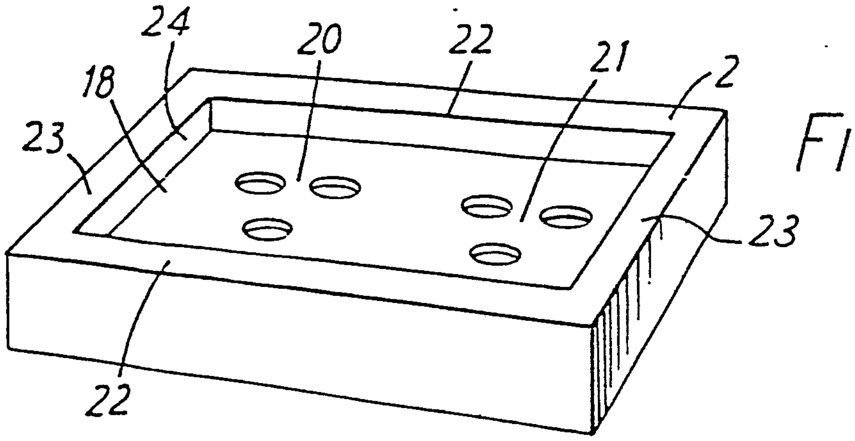

FIG. 8

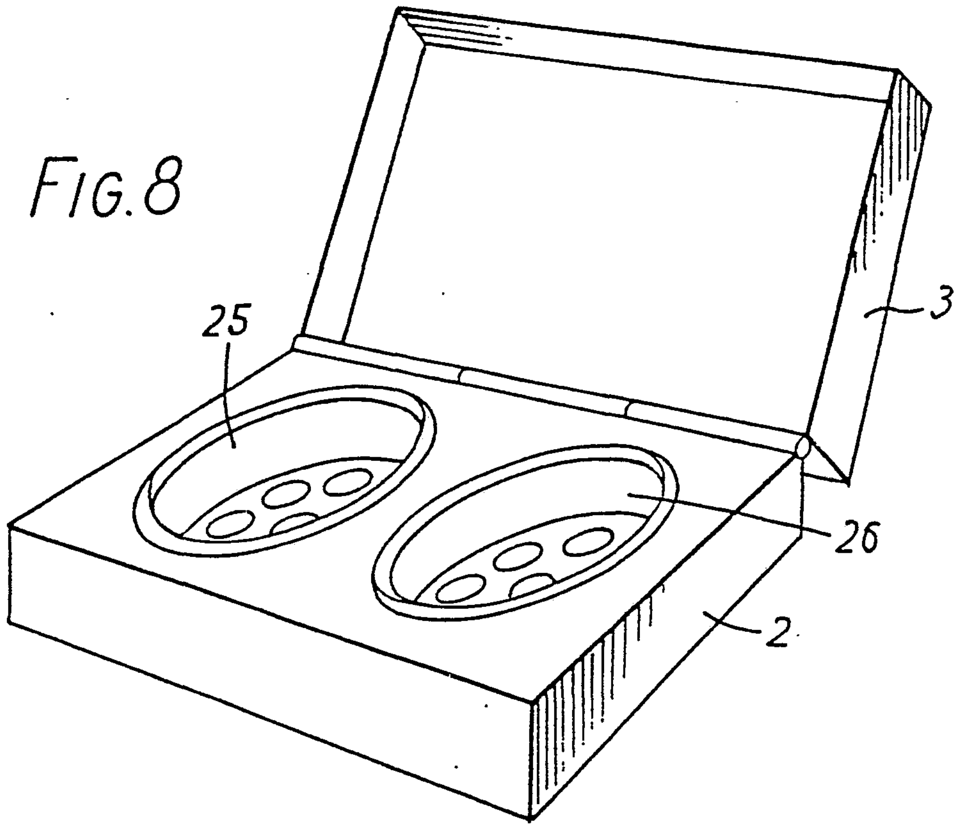


FIG. 9

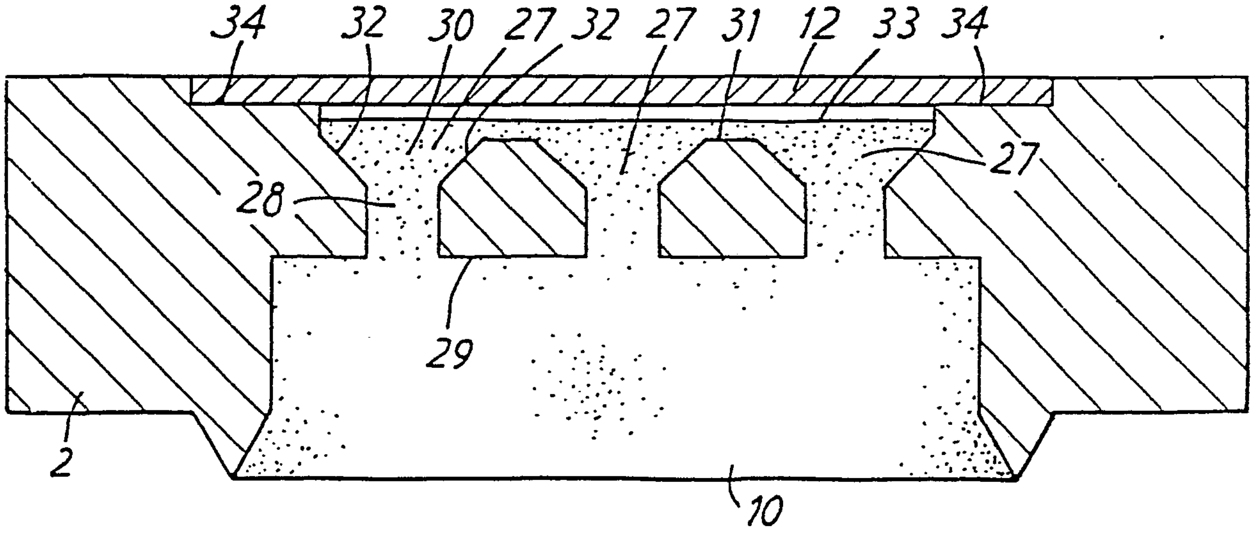

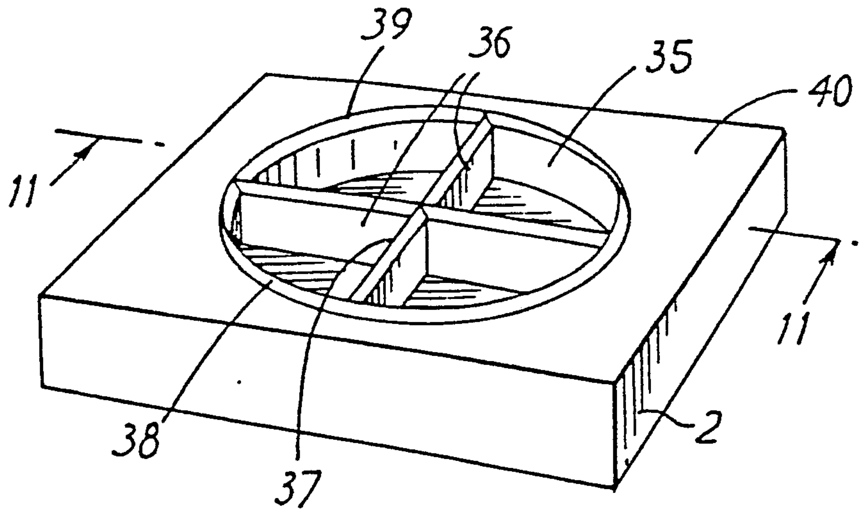


FIG.10

FIG.11

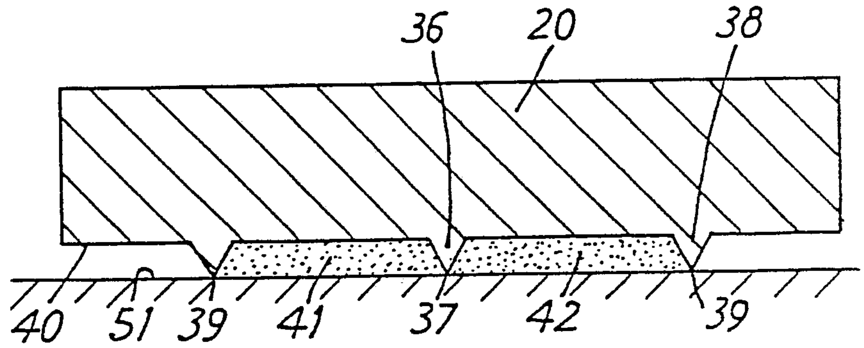


FIG.12